# EUROPEAN PATENT SPECIFICATION

(11) **EP 1 270 019 B1**
(45) Date of publication and mention of the grant of the patent: **26.10.2005**
(21) Application number: 02254539.6
(22) Date of filing: 27.06.2002
(51) Int. Cl.: A61L 2/16, B08B 3/04, B08B 3/08, C14C 3/28

(54) **Method for removing aldehyde-based stains**
Methode zur Entfernung von Flecken verursacht durch Aldehyde
Méthode pour enlever les tâches causées par des aldéhydes

(30) Priority: 28.06.2001 US 894402
(43) Date of publication of application: 02.01.2003
(73) Proprietor: ETHICON, INC., Somerville, NJ 08876 (US)
(72) Inventor: Zhu, Peter, Irvine, CA 92604 (US); Hui, Henry, Laguna Niguel, CA 92677 (US); Feldman, Les, Calabasas Hills, CA 91301 (US)
(74) Representative: Mercer, Christopher Paul

(56) References cited:
- DE-A- 19 704 615
- US-A- 3 892 523

## Description

### BACKGROUND OF THE INVENTION

### Field of the Invention

The invention relates to a method for removing or lessening the severity of aldehyde-based stains, in particular, stains formed by reaction of aldehydes with amino-containing compounds.

### Description of Related Art

Aldehyde-based germicides are commonly used in the healthcare facilities to disinfect and sterilize medical devices. One related problem associated with the use of aldehyde-based germicides such as formaldehyde, glutaraldehyde and *ortho*-phthalaldehyde (OPA) that have been reacted with amino-containing compounds is that the surfaces and/or the material that come into contact with the neutralized aldehyde become stained. Also residual aldehyde on surfaces or absorbed into the materials such as plastic will cause staining later when exposed to amino-containing compounds. This is a problem in a hospital environment when sterile surfaces and/or materials that are stained can cause concern that the devices are not sterile. Such surfaces include but or not limited to bench tops, floor surfaces and medical devices (metal or plastic). Some OPA related stains are the results of formation of Schiff's bases which are dark in color. Although the staining in the hospital environment is very problematic, there are no good measures to solve the problem at present. This invention offers solutions to this problem.

### SUMMARY OF THE INVENTION

According to the present invention there is provided a method for removing stains formed from aldehyde-based germicides and an amino compound, as defined in the appendant claims.

Among the advantages of the invention is removal of aldehyde-based stains to no stain or at least to a stain of lessened severity or intensity. This invention is effective for smooth surfaces such as glass, aluminum, copper, brass and stainless steel. Also, the invention may be used in a variety of formats such as a kit useful for making or using solutions or gels.

Additional features, embodiments, and benefits of the invention will be evident in view of the detailed description presented below.

### DETAILED DESCRIPTION OF THE INVENTION

Commonly assigned and co-pending patent application USSN 09/321,964 discloses the neutralization of aldehdye with amino acid. The neutralized aldehyde product can be formed by reacting an amino group from an amino acid or proteins with an aldehyde group of aldehydes to produce N-substituted adducts (imines or Schiff's bases) as shown below.

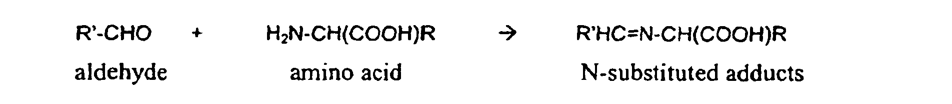

There are a variety of amino acids that are useful in neutralizing aldehydes. These amino acids include:
(1) Amino acids with apolar R groups (*e.g*., alanine, proline, amino-caproic acid, phenylalanine, tryptophan and methionine);
(2) Amino acids with polar R groups (*e.g.*, glycine, serine, cysteine, tyrosine, and glutamine);
(3) Amino acids with charged R groups (*e.g.*, aspartic acid, glutamic acid, lysine, arginine, and histidine); and,
(4) Peptides/polypeptides formed by any number or any type of amino acids and proteins.

The aldehyde can be a germicide that includes formaldehyde, glutaraldehyde, and OPA.

While the discovery of forming neutralized aldehydes for lessening the toxic effects of disposing of aldehyde treated waste was a major advance, resulting colored products (and hence susceptibility to forming stains) and the possibility of the neutralized aldehydes in reforming aldehydes under acidic conditions posed a problem in effectively maintaining nonhazardous waste because of the toxic effects of unneutralized aldehyde. The reversible reaction is depicted below for treatment of ASP-0027 glutaraldehyde **(1)** and *o*-phthaladehyde **(4)** with the amino acid, glycine **(2)** to the neutralized products, **(3)** and **(5)**, respectively:

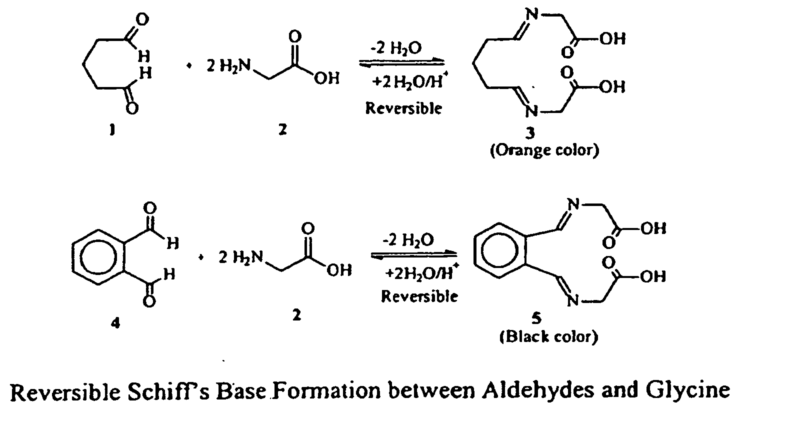

Commonly assigned and co-pending patent application USSN 09/747,230 discloses that the treatment of the neutralized products with a reducing agent to form amino acids does not cause them to revert back to unneutralized aldehyde. This reaction is depicted below for saturated moieties **(6)** and **(7)** as for the reduction of Schiff's bases **(3)** and **(5)** treated with the reducing agent sodium borohydride, NaBH₄:

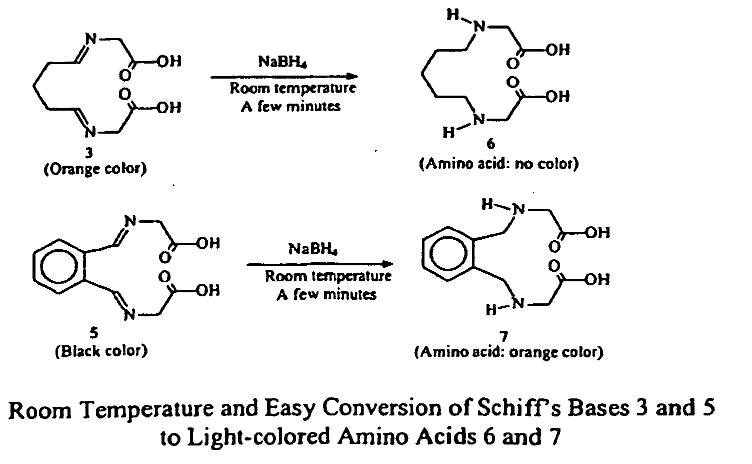

Being simple amino acids compounds **(6)** and **(7)** would be expected to be biodegradable and thus have significant benefit for the environment. This appears apparent by examination of the resemblance of the structures **(6)** and **(7)** with the natural essential amino acid proline, **(8)**. The corresponding resemblance is depicted with bold-faced highlighting of compounds **(6)** and **(7)** shown below:

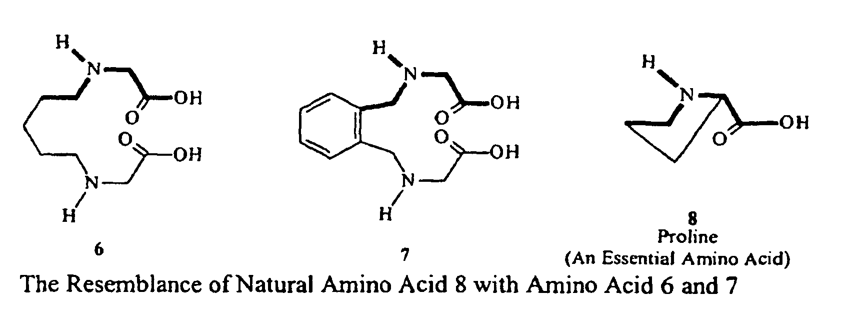

In contrast, Schiff's base **(3)** and **(5)** do not have the above characteristics and are likely very different compounds. One skilled in the art would suspect Schiff's bases to be harder to degrade in nature than the corresponding amino acids. For example, a piece of animal skin could decay in a few days in the wild while men's belts, made from animal skin too, could take many years. This is because the belt (leather) has undergone a tanning process. Tanning processes often employ the glutaraldehyde derivatives, such as depicted as structures **(9)** and **(10)** below, to cross-link proteins (Ref. a. Fein, M. L. and Filachione, E. M., "Tanning studies with aldehydes", *J. Am. Leather Chem. Assoc.*, **52,** 17, **1957;** b. Weligsberger, L. and Sadlier, C., "New developments in tanning with aldehydes", *J. Am. Leather Chem. Assoc.*, **52**, 2, **1957**; c. Hopwood, D., "Comparison of crosslinking abilities of glutaraldehyde, formaldehyde, and α-hydroxyadipaldehyde with bovine serum albumin and casein", *Histochemie,* **17**, 151, **1969).** It is well known that OPA has very similar protein cross-liking properties.

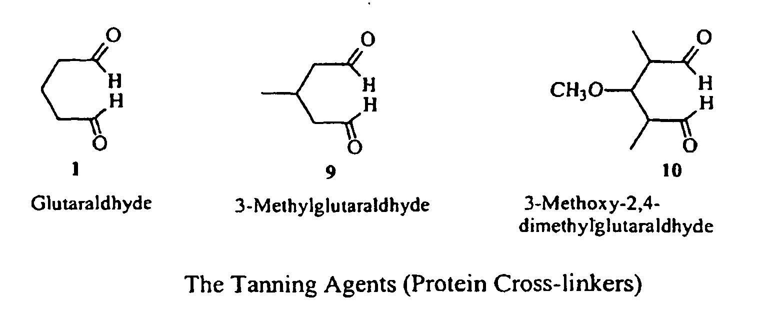

The conditions for Schiff's base reduction (and hence the removal or lessening of stains) is easy and convenient. Normally, it involves the mixing of the reducing agent, such as NaBH₄, and the imine, such as neutralized aldehyde, in a protonic solvent, such as water, ethanol, methanol, acid, or gel at room temperature. The acid may be organic or inorganic having active hydrogens. Due to the formation of colorless or light-colored products between the reducing agent and the imine, it is found that the reducing agent is a good chemical for removing the stains. The reducing agent can be applied to the stains by washing, spraying or dropping a solution containing the reducing agent onto the stained surface, by soaking the stained material in the solution containing the reducing agent, or by rubbing a gel or lotion comprising the reducing agent onto the stained surface or into the stained material.

The imine can be reduced by many reducing agents, such as LiAlH₄ (lithium aluminum hydride), NaBH₄ (sodium borohydride), NaCNBH₃ (sodium cyanoborohydride), Na-EtOH (metallic sodium in ethyl alcohol), and H₂/catalyst (hydrogen with a catalyst). A preferred reducing agent is NaBH₄.

### EXAMPLES

In the following examples, all percentages (%) referring to solutions are expressed as wt/vol, except in the case of Cidex® OPA where the percentages is expressed as wt/wt. The pH of the 1% aqueous sodium borohydride solution used in the following examples was measured to be 10.8.

### Example 1

(a) Cidex® OPA solution (0.55%, 1.0 mL) and glycine solution (1%, 1.0 mL) were mixed in a vial for 2 minutes and a green color developed and darkened quickly.
(b) Sodium borohydride solution (1%, 1.0mL) was added, shaken and mixed to the solution. The solution gradually became yellow and never darkened. The solution never turned black. Thus, the severity or intensity of the staining solution of (a) was lessened by the reducing agent sodium borohydride.

### Example 2

(a) Cidex® OPA solution (0.55%, 0.05mL) was applied to porous nylon film for about 10 minutes at room temperature (until dry). Glycine (1%, 0.05mL) was applied on the top of the Cidex® OPA spot. In about 10 minutes, the spot showed a green-black color.
(b) Sodium borohydride solution (1%, 0.15mL) was applied onto the top of the green-black spot. The green-black spot was de-stained immediately to turn to a much lighter color (yellowish brown).

### Example 3

(a) Cidex® OPA (0.55%, 0.05mL) was applied on porous nylon film and dried in about 2 hours at room temperature. Glycine (1%, 0.05mL) was added on the top of the Cidex® OPA spot and after 2 minutes, the spot became green-black.
(b) The stained nylon film was then soaked in sodium borohydride solution (1%, 10 mL) for 10 minutes, the green-black spot changed to a light-yellow spot. The color was noted to continue to fade with continued soaking.
(c) For comparison, the same experiment was repeated. However, instead of soaking in the sodium borohydride solution, the film was soaked in the water for 10 minutes. No reduction of the green-black color was observed. In fact, the green-black color never faded by soaking in water for 2 days.

### Example 4

(a) Cidex® OPA solution (0.55%, 0.05mL) was applied to filter paper ("Fisherbrand", qualitative P2, porosity: fine) and dried at room temperature for two hours. Glycine (1%, 0.05mL) was added on the top of the Cidex® OPA spot and after about 2 minutes, the spot became a green-black color.
(b) The stained filter paper was soaked in sodium borohydride solution (1%, 10 mL) for 5 minutes when the green-black spot changed to very light-yellow spot. The spot became colorless when the soaking continued for 2 hours.
(c) For comparison, the same experiment was repeated. However, instead of soaking in sodium borohydride solution, the filter paper was soaked in the water for 10 minutes. No reduction of the green-black color was observed. In fact, the green-black color never faded even after soaking in water for 2 days.

### Example 5

(a) Cidex® OPA solution (0.55%, 0.05mL) was applied to two (2) aluminum coupons (1x1 inches) and glycine (1%, 0.05mL) was added on the top of the Cidex® OPA spots. The aluminum coupons were then heated at 80°C for 2 hours. A brown-black residual spot formed on each coupon.
(b) One coupon was then soaked in sodium borohydride solution (1%, 10 mL) at room temperature. Surprisingly, the black residue on the aluminum surface disappeared instantly (less than 3 seconds). The solution became clear yellow. The surprisingly good result on the metal surface was not expected.
(c) The other coupon, the control coupon, was soaked in 10 mL water, where most of the brown-black residue dissolved gradually. However, there was still residue left on the coupon after 3 hours. The solution became dark green with black residue deposit on the bottom of the vial.

### Example 6

Example 5 was duplicated with brass coupons with even better results. The brown-black residue came off instantly while that on the control coupon came off only partially and the remainder came off very slowly and it remained even after soaking in water for 2 days.

### Example 7

Example 5 was duplicated with copper coupons. The same excellent result was observed as in Example 5.

### Example 8

Example 5 was duplicated with stainless steel surfaces. Thee same excellent result was observed as in Example 5.

### Example 9

(a) Cidex® OPA solution (0.55%, 0.05mL) was added into a scintillation vial and glycine (1%, 0.05mL) was added and mixed. The solution was heated at 80°C for 1 hour and a black residue was left on the bottom of the vial.
(b) Sodium borohydride solution (1%, 2 mL) was then added and the black residue was dissolved instantly to give a clear yellowish-brown solution (no green or black color).
(c) On the other hand, for the control experiment, the same procedure as above was followed except water (instead of sodium borohydride solution) was added to the vial. The black residue came off the glass wall after shaking and most of the residues remain suspended in the solution for 2 days. The solution appeared to be dark-green in color.

### Example 10

Examples 5-9 were repeated. In this case, fresh sodium borohydride versus aged solution (1 day old) was compared. All gave good results. All the black Schiff's base residue on different metal coupons came off instantly after exposing to the 1% sodium borohydride solution.

### Example 11

(a) Silicone tubing (OD = 0.60cm, wall thickness = 2mm) was soaked in the Cidex® OPA solution for seven days, washed with detergent, rinsed with water and then soaked in an amino solution to pick up the homogenous dark stains.
(b) The above silicone tubing (1.0cm in length) was soaked in sodium borohydride (1%, 5mL) for 2 hours, the majority of the color disappeared.
(c) Comparatively, the tubing soaked in water in place of the sodium borohydride did not fade in 1 month.

Therefore, based on Examples 1-11, it would be appreciated by those skilled in the art that depending on the nature of the material, the time or the efficiency to remove the stain may vary. Metal may require less time than non-metal non-absorbent or non-porous materials.

### Example 12

Example 11 was duplicated with a slight modification. Instead of water, 50% ethanol in water was used as the solvent. In this case, compared to the control (water as solvent), the de-staining rate is somewhat faster.

### Example 13

(a) Procedure: Cidex® OPA solution (0.55%, 10mL) was mixed with glycine (1%, 10mL). The mixed solution separately was applied to brass and aluminum coupons (20µL mixed solution for each stain). The coupons were then heated at 80°C for 2 hours. Brown-black residual spots formed.
(b) Each coupon (with one stain spot on it) was soaked in aqueous NaBH₄ solutions of varying concentrations as shown in Table 1. The time it took to remove the stain spot completely was recorded in Table 1.

**Table 1:**

| Effect of concentration on the Time (in seconds) required to remove Schiff based stains | | | | | |
|---|---|---|---|---|---|
| NaBH₄ Concentration | 1% | 0.5% | 0.25% | 0.125% | 0.0625% |
| Schiff base stain on brass | 2"±0.5 | 3"±1 | 4"±1 | 15"±5 | 26"±7 |
| Schiff' base stain on aluminum | 3"±0.5 | 3"+1 | 4"+1 | 12"±5 | 22"±7 |

Thus aqueous NaBH₄ solutions of various concentrations are quite effective for rapid removal of Schiff-based stains

### Example 14

### Reduction to product formats.

The following solutions could be used for soaking medical devices or wiping, or spraying medical surfaces or bench tops, floor surfaces etc. Since the pH is around 10.8, normal care should be taken (gloves/ventilation).

### Reduction to product format - (14-1):

Sodium borohydride powder (0.5-5g) is packed in bottles. Cap well. Add water before use (to dilute to near 1%).

### Reduction to product format - (14-2):

Sodium borohydride powder (0.5-5g) is packed in bottles. Cap well. Add mixture of water or an alcohol (ethanol, or isopropanol or methanol or mixed alcohol) before use (to dilute to near 1%).

### Reduction to product format - (14-3):

Sodium borohydride powder (0.5-5g) and an amino acid (such as glycine, lysine etc) are packed in bottles as dry powder. Cap well. Add mixture of water or an alcohol (ethanol, or isopropanol or methanol or mixed alcohol) before use (to dilute to near 1%).

### Reduction to product format - (14-4):

A container in the form of a spray bottle or bottle with a dropper may be used for formats 14-1 to 14-3.

### Reduction to product format - (14-5):

Instead of using bottle container, the sodium borohydride powder could be packed into aluminum bags or other types of bags to reduce product sizes.

### Reduction to product format - (14-6):

Nitrogen or another inert gas could be filled into the packaging formats in order to increase shelf life.

### Reduction to product format - (14-7):

Desiccators could be used for the above packaging formats in order to increase shelf life.

### Reduction to product format - (14-8):

Other reducing agents of similar nature could be used, such as sodium cyanoborohydride, metallic sodium in ethyl alcohol, and H₂/catalyst.

The invention is well suited for formation of kits wherein the dry powder form of the reducing agent may be combined with a solvent such as water, alcohol, or mixtures thereof, to form a solution of desired concentration and pH. Instructions within the kit would provide guidance regarding the proper amount of reducing agent to be combined with the solvent in order to provide a solution of effective strength to remove or lessen aldehyde-based stains. The resulting solutions may be used as soaking, wiping, or spraying solutions to treat the stained surfaces, for example, of medical devices, bench or table tops and floors.

## Claims

1. A method for removing a stain formed from an aldehyde-based germicide and an amino compound on the smooth surface of a non-absorbent or non-porous material comprising applying a reducing agent to the stained surface.

2. The method of claims 1, wherein the reducing agent is selected from the group consisting of LiAlH4, NaBH4, NaCNBH3, Na-Ethyl alcohol, and hydrogen/catalyst and mixtures thereof.

3. The method of claim 1, wherein the aldehyde-based stain comprises an imine formed from an aldehyde and a chemical containing amino group.

4. The method of claim 3, wherein the aldehyde comprises one or more (-CHO) functional group.

5. The method of claim 3, wherein the aldehyde comprises a dialdehyde.

6. The method of claim 3, wherein the aldehyde is selected from the group consisting of ortho-phthalaldehyde, glutaraldehyde, formaldehyde and mixtures thereof.

7. The method of claim 3, wherein the chemical is selected from the group consisting of alkyl amine, aryl amine, polymer amine, protein, peptide, amino acid, and any combination thereof.

8. The method of claim 1, wherein the surface or material is selected from the group consisting of a bench top, floor; or medical device.

## Patentansprüche

1. Verfahren zur Entfernung eines Flecks, der durch ein Aldehyd-basiertes Germizid und einer Aminoverbindung auf der glatten Oberfläche eines nicht-absorbierenden oder nichtporösen Materials verursacht wurde, umfassend die Auftragung eines reduzierenden Mittels auf die gefärbte Oberfläche.

2. Verfahren nach Anspruch 1, wobei das reduzierende Mittel ausgewählt ist aus der Gruppe bestehend aus LiAlH4, NaBH4, NaCNBH3, Na-Ethylalkohol, und Wasserstoff/Katalysator und Gemische davon.

3. Verfahren nach Anspruch 1, wobei der Aldehyd-basierte Fleck ein Imin umfaßt, gebildet durch ein Aldehyd und einer Chemikalie enthaltend eine Aminogruppe.

4. Verfahren nach Anspruch 3, wobei das Aldehyd eine oder mehrere (-CHO) funktionelle Gruppen umfaßt.

5. Verfahren nach Anspruch 3, wobei das Aldehyd ein Dialdehyd umfaßt.

6. Verfahren nach Anspruch 3, wobei das Aldehyd ausgewählt ist aus der Gruppe bestehend aus ortho-Phthalaldehyd, Glutaraldehyd, Formaldehyd und Gemischen davon.

7. Verfahren nach Anspruch 3, wobei die Chemikalie ausgewählt ist aus der Gruppe bestehend aus Alkylamin, Arylamin, Polymeramin, Protein, Peptid, Aminosäure und jeder Kombination davon.

8. Verfahren nach Anspruch 1, wobei die Oberfläche oder das Material ausgewählt ist aus der Gruppe bestehend aus einem Labortisch, Fußboden, oder einer medizinischen Vorrichtung.

## Revendications

1. Procédé pour enlever une tâche formée par un germicide à base d'aldéhyde et un composé amino sur la surface lisse d'un matériau non absorbant ou non poreux comprenant l'application d'un agent réducteur sur la surface tâchée.

2. Procédé selon la revendication 1, dans lequel l'agent réducteur est choisi dans le groupe constitué par LiAlH4, NaBH4, NaCNBH3, l'alcool de Na-éthyle et un catalyseur/hydrogène et leurs mélanges.

3. Procédé selon la revendication 1, dans lequel la tâche à base d'aldéhyde comprend une imine formée à partir d'un aldéhyde et d'un produit chimique contenant un groupe amino.

4. Procédé selon la revendication 3, dans lequel l'aldéhyde comprend un ou plusieurs groupe(s) fonctionnel(s) (-CHO).

5. Procédé selon la revendication 3, dans lequel l'aldéhyde comprend un dialdéhyde.

6. Procédé selon la revendication 3, dans lequel l'aldéhyde est choisi dans le groupe constitué par l'ortho-phtalaldéhyde, le glutaraldéhyde, le formaldéhyde et leurs mélanges.

7. Procédé selon la revendication 3, dans lequel le produit chimique est choisi dans le groupe constitué par une alkyl-amine, une aryl-amine, une amine polymère, une protéine, un peptide, un acide aminé et leurs combinaisons quelconques.

8. Procédé selon la revendication 1, dans lequel la surface ou le matériau est choisi dans le groupe constitué par le dessus d'un banc, le sol ou un dispositif médical.
